(19) Europäisches Patentamt
European Patent Office
Office européen des brevets

(11) Publication number: 0 374 089 A2

(12) # EUROPEAN PATENT APPLICATION

(21) Application number: 89810846.9

(51) Int. Cl.5: A61K 37/43, A61K 37/02

(22) Date of filing: 08.11.89

The title of the invention has been amended (Guidelines for Examination in the EPO, A-III, 7.3).

(30) Priority: 11.11.88 DE 3838380

(43) Date of publication of application: 20.06.90 Bulletin 90/25

(84) Designated Contracting States: ES

(71) Applicant: SANDOZ AG
Lichtstrasse 35
CH-4002 Basel(CH)

(72) Inventor: Chrubasik, Joachim
Schöneckstrasse 13
D-7800 Freiburg(DE)
Inventor: Chrubasik, Sigrun
Schöneckstrasse 13
D-7800 Freiburg(DE)

(54) **Use of somatostatin derivatives as lung protecting agents.**

(57) Somatostatin and analogues and derivatives thereof in free form or in pharmaceutically acceptable salt or complex form are useful for protecting the lungs, e.g. for the treatment of adult respiratory distress syndrome.

EP 0 374 089 A2

# LUNG PROTECTING AGENTS

The present invention relates to a new use, in particular a new use for the compound group comprising the naturally occurring somatostatin and somatostatin analogues and derivatives, in free form or in pharmaceutically acceptable salt or complex form, said compound group being referred to hereinafter collectively as COMPOUNDS OF THE INVENTION.

Somatostatin is a tetradecapeptide incorporating a cyclic dodecapeptide having the structure

H-Ala-Gly-Cys-Lys-Asn-Phe-Phe-Trp-Lys-Thr-Phe-Thr-Ser-Cys-OH
1 2 3 4 5 6 7 8 9 10 11 12 13 14

and has the properties of inhibiting the release of growth hormone, insulin and glucagon and reducing gastric secretions.

By the terms "somatostatin analogue or derivative" as used herein is meant any straight-chain or cyclic polypeptide derived from that of the naturally occurring tetradecapeptide somatostatin wherein one or more amino acid units have been omitted and/or re placed by one or more other amino radical(s) and/or wherein one or more functional groups have been replaced by one or more other functional groups and/or one or more groups have been replaced by one or several other isosteric groups. The terms "analogue or derivative" also include the corresponding peptides bearing a sugar residue. In general, the term covers all modified derivatives of a biologically active peptide which exhibit a qualitatively similar effect to that of the unmodified somatostatin peptide, e.g. they bind to somatostatin receptors and inhibit secretion of GH.

Cyclic, bridge cyclic and straight-chain somatostatin analogues or derivatives are known and have been described together with processes for their production e.g. in US Patent Specifications 4,310,518 and 4,235,886, in European Patent Specifications EP-A-1295; 29,310; 29,579; 63,308; 70,021; 215,171; 203,031; 214,872; 143 307; 298,732; 277,419 and in Belgian Patent Specification BE-A-900,089.

When the COMPOUNDS OF THE INVENTION bear a sugar residue, this is preferably coupled to an amino group thereof by a coupling other than a direct N-glycosidic bond, preferably to a N-terminal amino group and/or to at least one amino group present in a peptide side chain, more preferably to a N-terminal amino group. Such compounds and their preparation are disclosed e.g. in WO 88/02756.

Preferred compounds of the invention are:

A. Naturally occurring somatostatin.

B. Compounds of formulae I to III

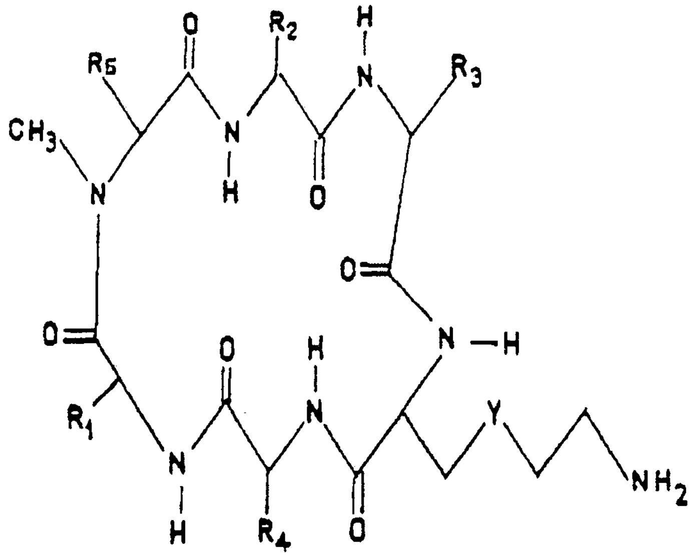

(I)

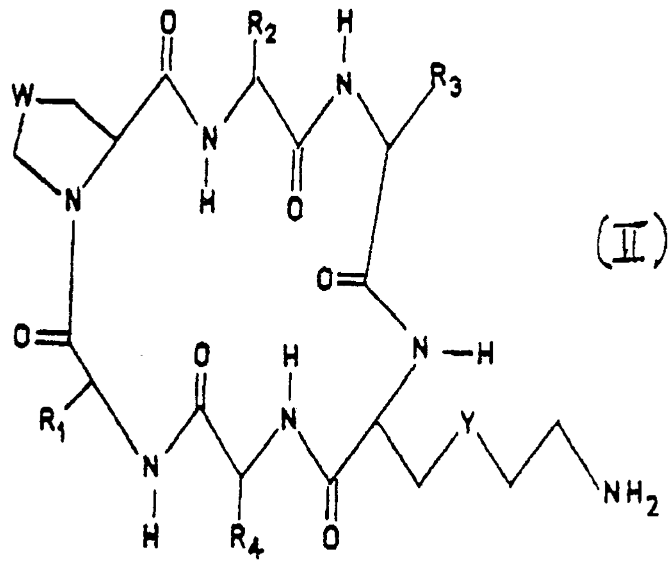

(II)

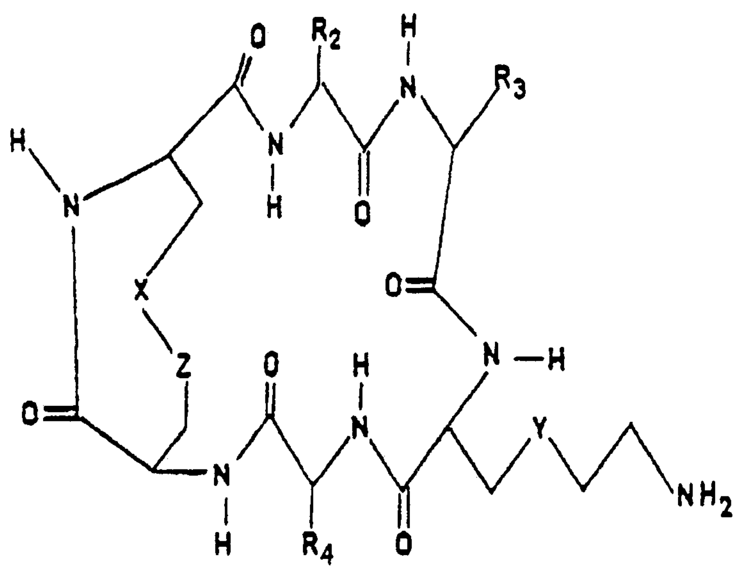

(III)

wherein
W is S or $(CH_2)_s$ where s is 0, 1 or 2;
one of X and Z is S and the other is S or $CH_2$;
Y is S or $(CH_2)_t$ where t is 0, 1 or 2;
each of $R_1$ and $R_2$ independently of the other, is $C_{1-5}$ alkyl, benzyl, benzyl having one or two $C_{1-5}$ alkyl, halogen, hydroxy, amino, nitro, and/or $C_{1-5}$ alkoxy substituents, or $C_{1-5}$ alkyl substituted with a 5- or 6-membered heterocyclic ring;
$R_3$ is 3-indolylmethyl, either unsubstituted or having $C_{1-5}$ alkyl, $C_{1-5}$ alkoxy or halogen substitution;
$R_4$ is $C_{1-5}$ alkyl, $C_{1-5}$ hydroxyalkyl, benzyl, carboxy-($C_{1-5}$ alkyl), amino ($C_{1-5}$ alkyl) or benzyl having a $C_{1-5}$ alkyl, halogen, hydroxy, amino, nitro and/or $C_{1-5}$ alkoxy substituent;
$R_5$ is $C_{1-5}$ alkyl, benzyl, or benzyl having a $C_{1-5}$ alkyl, halogen, hydroxy, amino, nitro, and/or $C_{1-5}$ alkoxy substituent.

Examples of $C_{1-5}$ alkyl groups are methyl, ethyl, propyl, *iso*-propyl, butyl, *sec*-butyl and pentyl; examples of $C_{1-5}$ alkoxy groups are methoxy, ethoxy, propoxy, isopropoxy, butoxy, *tert*-butoxy, and pentoxy; halogens are fluorine, chlorine, bromine, or iodine: and the term "5- or 6-membered heterocyclic ring" represents such rings with one or two oxygen, nitrogen and/or suphur heteroatoms, e.g. imidazole, furan, thiazole, pyrazole and pyridine.

In the compounds of formulae I, II and III, there are several asymmetric centres which lead to the existence of optical isomers for such compounds. For each of the asymmetric centres of the various amino acids which make up these cyclic hexapeptides, both the D and L configurations are included.

The following are representative cyclic hexapeptide analogues of somatostatin of Formulae I, II and III:

```
N-Me-Ala-Phe-Trp
      |       |
      Phe-Thr-Lys
```

Ia

```
Pro-Phe-Trp
|       |
Phe-Thr-Lys
```

IIa

```
 ------Cys-Phe-Trp
|        |     |
S        |     |
|        |     |
S        |     |
|        |     |
 ------Cys-Thr-Lys
```

IIIa

Preferred Formula I compounds are:
1) Cyclo-(N-Me-Ala-Tyr-D-Trp-Lys-Thr-Phe)
2) Cyclo-(N-Me-Ala-Phe-D-Trp-Lys-Thr-Phe)
3) Cyclo-(N-Me-Ala-Phe-L-Trp-Lys-Thr-Phe)
4) Cyclo-(N-Me-Ala-Phe-D-Trp-Lys-Thr-p-Cl-Phe)
5) Cyclo-(N-Me-Ala-Phe-D-S-F-Trp-Lys-Thr-Phe)
6) Cyclo-(N-Me-Ala-Phe-L-S-F-Trp-Lys-Thr-Phe)

7) Cyclo-(N-Me-Ala-Phe-D-Trp-Lys-Ser-Phe)
8) Cyclo-(N-Me-Ala-Tyr-D-Trp-Lys-Val-Phe)
9) Cyclo-(N-Me-Ala-Tyr-D-Trp-Lys-Val-Trp)
10) Cyclo-(N-Me-Ala-Tyr-L-Trp-Lys-Val-Phe)
11) Cyclo-(Ser-Ala-N-Me-Phe-His-D-Trp-Lys)

Preferred Formula II compounds are:

12) Cyclo-(Pro-Tyr-D-Trp-Lys-Thr-Phe)
13) Cyclo-(Pro-Phe-D-Trp-Lys-Thr-Phe)
14) Cyclo-(Pro-Phe-L-Trp-Lys-Thr-Phe)
15) Cyclo-(Pro-Phe-D-Trp-Lys-Thr-p-Cl-Phe)
16) Cyclo-(Pro-Phe-D-5-F-Trp-Lys-Thr-Phe)
17) Cyclo-(Pro-Phe-L-5-F-Trp-Lys-Thr-Phe)
18) Cyclo-(Pro-Phe-D-Trp-Lys-Ser-Phe)

Preferred Formula III compounds are:

19) Cyclo-(Cys-Cys-Tyr-D-Trp-Lys-Thr)

20) Cyclo-(Cys-Cys-Tyr-D-Trp-Lys-Val)

21) Cyclo-(Cys-Cys-Tyr-L-Trp-Lys-Val)

22) Cyclo-(Cys-Cys-Phe-D-Trp-Lys-Thr)

23) Cyclo-(Cys-Cys-Phe-L-Trp-Lys-Thr)

24) Cyclo-(Cys-Cys-His-D-Trp-Lys-Thr)

25) Cyclo-(Cys-Cys-His-D-Trp-Lys-Val)

26) Cyclo-(Cys-Cys-Ala-Phe-D-Trp-Lys-Thr).

C. Compounds of formula IV,

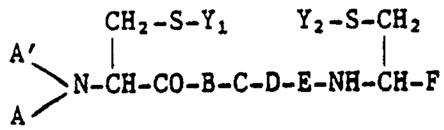

IV

wherein

A is $C_{1-12}$ alkyl, $C_{7-10}$ phenylalkyl or a group of formula RCO-, whereby

i) R is hydrogen, $C_{1-11}$ alkyl, phenyl or $C_{7-10}$ phenylalkyl, or

ii) RCO-is

a) an L- or D-phenylalanine residue optionally ring-substituted by halogen, $NO_2$, $NH_2$, OH, $C_{1-3}$ alkyl and/or $C_{1-3}$ alkoxy

b) the residue of a natural or a synthetic α-amino acid other than defined under a) above or of a corresponding D-amino acid, or

c) a dipeptide residue in which the individual amino acid residues are the same or different and are selected from those defined under a) and/or b) above, the α-amino group of amino acid residues a) and b) and the N-terminal amino group of dipeptide residues c) being optionally mono-or di-$C_{1-12}$ alkylated or substituted by $C_{1-8}$alkanoyl,

A′ is hydrogen or, when A is $C_{1-12}$ alkyl or $C_{7-10}$ phenylalkyl, also $C_{1-12}$ alkyl or $C_{7-10}$ phenylalkyl,

$Y_1$ and $Y_2$ represent together a direct bond or

each of $Y_1$ and $Y_2$ is independently hydrogen or a radical of formulae (1) to (5)

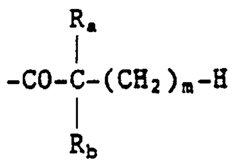

(1)

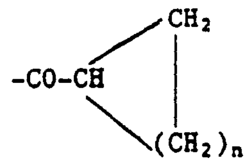

(2)

$-CO-NHR_c$

(3)

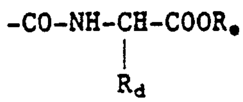

(4)

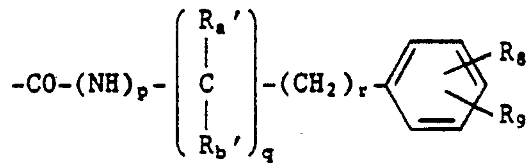

(5)

wherein $R_a$ is methyl or ethyl
$R_b$ is hydrogen, methyl or ethyl
m is a whole number from 1 to 4
n is a whole number from 1 to 5
$R_c$ is $(C_{1-6})$alkyl
Rd represents the substituent attached to the α-carbon atom of a natural or synthetic α-amino acid (including hydrogen)
$R_e$ is $(C_{1-5})$alkyl
$R_a'$ and $R_b'$ are independently hydrogen, methyl or ethyl,
$R_8$ and $R_9$ are independently hydrogen, halogen, $(C_{1-3})$alkyl or $(C_{1-3})$alkoxy,
p is 0 or 1,
q is 0 or 1, and
r is 0, 1 or 2,
B is -Phe- optionally ring-substituted by halogen, $NO_2$, $NH_2$, OH, $C_{1-3}$alkyl and /or $C_{1-3}$alkoxy , or 3-(2-naphthyl)-alanine
C is (L)-Trp- or (D)-Trp- optionally α-N-methylated and optionally benzene-ring-substituted by halogen, $NO_2$, $NH_2$, OH, $C_{1-3}$ alkyl and/or $C_{1-3}$ alkoxy
D is Lys, Lys in which the side chain contains O or S in β-position, γF-Lys, δF-Lys, optionally α-N-methylated, or a 4-aminocyclohexylAla or 4-aminocyclohexylGly residue
E is Thr, Ser, Val, Phe, Tyr, Ile or an aminoisobutyric or aminobutyric acid residue
F is $-COOR_7$, $-CH_2OR_{10}$,

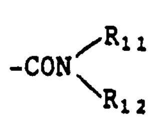

or

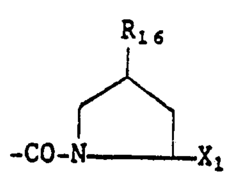

wherein
$R_7$ is hydrogen or $C_{1-3}$alkyl,
$R_{10}$ is hydrogen or the residue of a physiologically acceptable, physiologically hydrolysable ester,
$R_{11}$ is hydrogen, $C_{1-3}$alkyl, phenyl or $C_{7-10}$phenyl-alkyl,
$R_{12}$ is hydrogen, $C_{1-3}$alkyl or a group of formula-$CH(R_{13})-X_1$,
$R_{13}$ is $CH_2OH$, $-(CH_2)_2-OH$, $-(CH_2)_3-OH$, or $-CH(CH_3)OH$ or represents the substituent attached to the α-carbon atom of a natural or synthetic α-amino acid (including hydrogen) and
$X_1$ is a group of formula $-COOR_7$, $-CH_2OR_{10}$ or

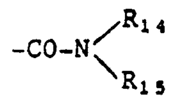

wherein
$R_7$ and $R_{10}$ have the meanings given above,
$R_{14}$ is hydrogen or $C_{1-3}$alkyl and
$R_{15}$ is hydrogen, $C_{1-3}$alkyl, phenyl or $C_{7-10}$phenylalkyl, and
$R_{16}$ is hydrogen or hydroxy,
with the proviso that
when $R_{12}$ is -CH($R_{13}$)-$X_1$ then $R_{11}$ is hydrogen or methyl,
wherein the residues B, D and E have the L-configuration, and the residues in the 2-and 7-position and any residues $Y_1$ 4) and $Y_2$ 4) each independently have the (L)- or (D)- configuration,
in free form or in pharmaceutically acceptable salt or complex form.

In the compounds of formula IV, the following significances are preferred either individually or in any combination or sub-combination:

1. A is $C_{7-10}$ phenylalkyl, especially phenethyl, or a group of formula RCO. Preferably A is a group of formula RCO.

1.1. Preferably R is $C_{1-11}$ alkyl or $C_{7-10}$ phenylalkyl, especially $C_{7-10}$ phenylalkyl, more especially phenethyl, or
RCO has the meanings a), b) or c).

1.2. When RCO has the meanings a), b) or c), the α-amino group of amino acid residues a) and b) and the N-terminal amino group of dipeptide residues c) is preferably non-alkylated or mono-$C_{1-12}$ alkylated, especially -$C_{1-8}$ alkylated, more especially -methylated. Most preferably the N-terminal is non-alkylated.

1.3. When RCO has the meaning a) this is preferably a′) an L-or D-phenylalanine or -tyrosine residue optionally mono-N-$C_{1-12}$ alkylated. More preferably a′) is an L- or D-phenylalanine residue or an L- or D-N-($C_{1-8}$-alkyl)-phenylalanine residue. Most preferably a′) is a D-phenylalanine or D-N-($C_{1-8}$ alkyl)-phenylalanine residue, especially a D-phenylalanine or D-(N-methyl)-phenylalanine residue.

1.4. When RCO has the meaning b) or c) the defined residue is preferably lipophilic. Preferred residues b) are thus b′) α-amino acid residues having a hydrocarbon side chain, e.g. alkyl with 3, preferably 4, or more C atoms, e.g. up to 7 C-atoms, naphthyl-methyl or heteroaryl, e.g. 3-(2- or 1-naphthyl)-alanine residue or tryptophane residue, said residues having the L- or D-configuration, and preferred residues c) are dipeptide residues in which the individual amino acid residues are the same or different and are selected from those defined under a′) and b′) above.

Example of a residue c) is e.g. 3-(2-naphthyl)-alanine residue.

1.5. Most preferably RCO has the meaning a) especially the meaning a′).

2. B is B′, where B′ is Phe or Tyr.

3. C is C′, where C′ is (D)Trp.

4. D is D′, where D′ is Lys, MeLys or Lys(ε-Me), especially Lys.

5. E is E′, where E′ is Val or Thr, especially Thr.

6. F is F′, where F′ is a group of formula

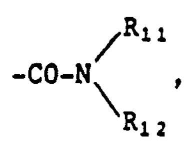

especially a group of formula

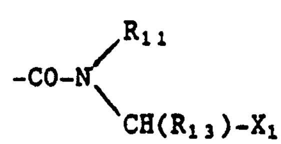

(in which case $R_{11}$ = H or $CH_3$). In the latter case the moiety -CH($R_{13}$)-$X_1$ preferably has the L-configuration.

6.1. $R_{11}$ is preferably hydrogen.

6.2. As the substituent attached to the α-carbon atom of a natural amino acid (i.e. of formula $H_2N$-CH-($R_{13}$)-COOH), $R_{13}$ is preferably $-CH_2OH$, $-CH(CH_3)-OH$, isobutyl or butyl, or $R_{13}$ is $-(CH_2)_2-OH$ or $-(CH_2)_3-OH$. It is especially $-CH_2OH$ or $-CH(CH_3)OH$.

6.3. $X_1$ is preferably a group of formula

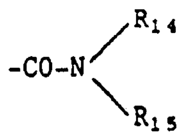

or $-CH_2-OR_{10}$, especially of formula $-CH_2-OR_{10}$ and $R_{10}$ is preferably hydrogen or has the meaning given under 7 below. Most preferably $R_{10}$ is hydrogen.

7. As the residue of a physiologically acceptable, physiologically hydrolysable ester $R_{10}$ is preferably HCO, $C_{2-12}$ alkylcarbonyl, $C_{8-12}$ phenylalkylcarbonyl or benzoyl.

8. Preferably the residues in the 2- and 7-positions have the L-configuration.

9. Preferably $Y_1$ and $Y_2$ together represent a direct bond.

Preferred compounds of formula IV are for example:

H-(D)Phe-Cys-Phe-(D)Trp-Lys-Thr-Cys-Thr-ol

also known as octreotide

(D)Phe-Cys-Tyr-(D)Trp-Lys-Val-Cys-ThrNH$_2$

(D)Phe-Cys-Tyr-(D)Trp-Lys-Val-Cys-TrpNH$_2$

(D)Trp-Cys-Phe-(D)Trp-Lys-Thr-Cys-ThrNH$_2$

(D)Phe-Cys-Phe-(D)Trp-Lys-Thr-Cys-ThrNH$_2$

β-Naphthyl-(D)Ala-Cys-Tyr-(D)Trp-Lys-Val-Cys-ThrNH$_2$

(D)Phe-Cys-Tyr-(D)Trp-Lys-Val-Cys-β-Nal-NH$_2$

β-Nal-Cys-Tyr-(D)Trp-Lys-Val-Cys-β-Nal-NH$_2$

(D)Phe-Cys-β-Nal-(D)Trp-Lys-Val-Cys-Thr-NH$_2$

A most preferred compound of formula IV is the compound IVa

H- (D)Phe-Cys-Phe-(D)Trp-Lys-Thr-Cys-Thr-ol

Suitable derivatives bearing at least one sugar residue are e.g. compounds of formula IV including the compound octreotide bearing a sugar residue. Such compounds are known and have been described together with processes for their production, e.g. in WO 88/02756, the contents of which being incorporated herein by reference.

Preferred sugar somatostatin derivatives are the compounds of formula IV which have a sugar residue on the N-terminal amino group, e.g. a residue of formula (a)

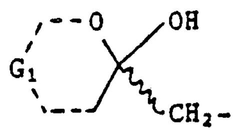

(a)

which is the deoxy residue of an aldose, e.g. a radical obtainable by means of an Amadori rearrangement from a natural or synthetically accessible mono-, di- or oligosaccharide, or
a residue of formula (b)

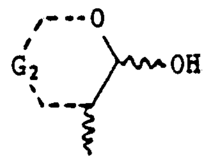

(b)

which is the deoxy residue of a ketose, e.g. a radical obtainable by means of a Heyns rearrangement from a natural or a synthetically accessible mono-, di- or oligoketose, or a residue of formula (c)

$G_3$-CO- (c)

wherein
$G_3CO$ is the residue of an uronic acid, e.g. glucuronic or galacturonic acid, or of a polyhydroxymono- or dicarboxylic acid, e.g. gluconic acid, glucaric acid, quinic acid, acetylmuranic acid, acetylneuraminic acid or D-glucosaminic acid, and
a residue of formula (d 1) to (d 4)

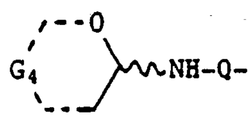

(d 1)

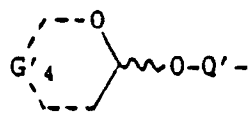

(d 2)

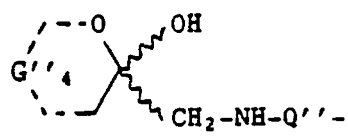

(d 3)

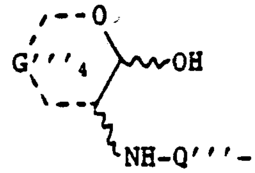

(d 4)

wherein
Q, Q$'$, Q$''$ and Q$'''$ are groups coupling the somatostatin peptide with the sugar residue, e.g. Q is preferably the radical of a dicarboxylic acid, $-C_bH_{2b}-CO-$(b is 1 to 6) or CO or CS, Q$'$ is preferably $-C_bH_{2b}-CO-$ or the radical of a dicarboxylic acid, each of -NHQ$''$ and -NHQ$'''$ is preferably the radical of a $\omega$-aminocarboxylic acid, e.g. $-NH-C_bH_{2b}-CO-$, and

$G_4$, $G'_4$, $G''_4$ and $G'''_4$ have the definitions given above for $G_1$ or $G_2$;
a residue of formula (e 1) or (e 2)

$HOCH_2$-$(CHOH)_c$-CYY-$CH_2$- (e 1)

$HOCH_2$-$(CHOH)_c$- CH-$CH_2OH$ (e 2)

wherein
one of Y is hydrogen and the other is hydrogen or hydroxy,
and
c is 2, 3 or 4,
wherein any one of the free hydroxy groups in the polyol moiety of (e 1) and (e 2) is optionally bound in glycosidic manner to a reducing mono-, di- or oligosaccharide or amino sugar.

Particularly preferred compounds are

$N^{\alpha}$-[α-glucosyl(1-4)-deoxyfructosyl]-DPhe-Cys-Phe-DTrp-Lys-Thr-Cys-Thr-ol

(referred to as compound of formula IVb) and

$N^{\alpha}$-β-deoxy-fructosyl-DPhe-Cys-Phe-DTrp-Lys-Thr-Cys-Thr-ol

(referred to as compound of formula IVc).

D. Compounds of formulae V to IX

H-Cys-Phe-Phe-(D)Trp-Lys-Thr-Phe-Cys-OH V

[see Vale et al., Metabolism, 27, Supp. 1, 139, (1978)]

Asn-Phe-Phe-(D)Trp-Lys-Thr-Phe-Gaba VI

[see European Patent Publication No 1295 and Application No. 78 100 994.9]

NMePhe-His-(D)Trp-Lys-Val-Ala VII

[see R.F. Nutt et al. Klin. Wochenschr. (1986) 64 (Suppl. VII) 71-73.

H-Cys-His-His-Phe-Phe-(D)Trp-Lys-Thr-Phe-Thr-Ser-Cys-OH VIII

(see EP-A-200,188)

Cyclo-(DTrp-Lys-Val-Phe-NMeAla-Tyr) IX

(see EP-A-70,021)

The contents of all the above publications including the specific compounds are specifically incorporated herein by reference.

The compounds of the invention may exist e.g. in free form, salt form or in the form of complexes thereof. Acid addition salts may be formed with e.g. organic acids, polymeric acids and inorganic acids. Such acid addition salt forms include e.g. the hydrochlorides and acetates. Complexes are e.g. formed from compounds of the invention on addition of inorganic substances, e.g. inorganic salts or hydroxides such as Ca- and Zn-salts, and/or an addition of polymeric organic substances.

Somatostatin and somatostatin analogues and derivatives are mainly disclosed as having an inhibitory

effect on growth hormone, glucagon and insulin secretion.

In accordance with the present invention it has now been found that COMPOUNDS OF THE INVENTION have lung protecting properties.

In accordance with the particular findings of the invention, the present invention provides, in a first aspect:

1. A method of protecting the lungs against acute lung injuries in a subject in need thereof, which method comprises administering to said subject an effective amount of a COMPOUND OF THE INVENTION.

1.1 A method of preventing or treating respiratory distress syndrome in a subject in need thereof, which method comprises administering to said subject an effective amount of a COMPOUND OF THE INVENTION.

1.2 A method of preventing or treating pulmonary damages induced by or associated with oxygen therapy in a subject in need thereof, which comprises administering to said subject an effective amount of a COMPOUND OF THE INVENTION.

1.3 A method for improving oxygen tolerability in a subject in need thereof, which comprises administering to said subject an effective amount of a COMPOUND OF THE INVENTION.

1.4 A method of oxygenating with $O_2$ at a concentration > 40 % in a subject in need of such a treatment which method comprises administering to said subject an effective amount of a COMPOUND OF THE INVENTION.

In a particular embodiment the invention provides a method as defined under 1 and 1.1 to 1.4 above for example for the treatment of acute respiratory failure, adult respiratory distress syndrome (ARDS), respiratory distress syndrome (hyaline membrane disease) in infants and newborns, severe pneumonia, oxygen pneumonitis, pulmonary edema, and further conditions and diseases requiring oxygenation assistance and monitoring over a prolonged period, for example at least 60 hours, with an oxygen concentration > 40 %, e.g. intra- or postoperative anesthesia.

In a further preferred embodiment the invention provides a method for preventing, inhibiting the development of or treating adult respiratory distress syndrome (ARDS) in a subject in need thereof, comprising administering to said subject an effective amount of a COMPOUND OF THE INVENTION. ARDS may be associated with or induced by a variety of acute processes that directly or indirectly injure the lung, e.g. septicemia, sepsis syndrome, bacterial or viral pneumonias, aspiration of gastric contents, massive trauma, brain trauma, direct chest trauma, prolonged or profond shock, burns, near-drowning, fat embolism, massive blood transfusion, cardiopulmonary bypass, $O_2$ toxicity, acute hemorrhagic pancreatitis or endotoxin shock.

As alternatives to the above the present invention also provides:

2. A COMPOUND OF THE INVENTION for use in any method as defined above;

3. A COMPOUND OF THE INVENTION for use in the manufacture of a pharmaceutical composition for use in any method as defined above;

4. A pharmaceutical composition for use in any method as defined above comprising a COMPOUND OF THE INVENTION together with one or more pharmaceutically acceptable diluents or carriers therefor.

Utility of the COMPOUNDS OF THE INVENTION in treating diseases and conditions as hereinabove specified, is indicated, e.g. in standard pharmacological test methods as well as in clinic, for example in accordance with the methods hereinafter described.

**A. Oxygen Toxicity**

12 days are anesthetized with 5 mg/kg thiopental and after intubation are kept anesthetized with thiopental sodium and pancuronium bromide. The rectal temperature is maintained between 37.5 to 38.5 ° C. The V. jugularis interna is cannulated and a Swan-Ganz catheter and a further catheter are introduced in A. femoralis. The following parameters are assessed:

- heart rate
- right atrial pressure
- systolic, diastolic and mean arterial pressure
- pulmonary capillary wedge pressure
- pulmonary arterial pressure
- tidal volume
- minute volume
- end expiratory pressure

- end pressure of expired $CO_2$
- end expiratory $CO_2$ concentration
- arterial and venous blood gases, oxygen saturation
- hemoglobin, electrolyte and glucose in serum.

The cardiac output is measured by means of the thermodilution method. The shunt volume and the dead space are evaluated from the above parameters. A catheter is placed in the contralateral V. jugularis for liquid substitution.

30 minutes after inducing anesthesia, the baseline values of above parameters are assessed. The dogs are divided in 2 groups: in one group which serves as a control the animals receive in the tubus for 48 hours a 5 l/min oxygen sprayed sodium chloride solution, and in the second group the animals are treated through the tubus for 48 hours with the same sodium chloride solution containing additionally the compound to be tested and sprayed with 5 l/min oxygen.

The above parameters are assessed at intervals of 4 hours. The observed differences between baseline and subsequent assessments are compared with the control animals and analyzed. At the end of the treatment lung tissues of the control and treated dogs are submitted to histological examination.

In this test, on administration of a COMPOUND OF THE INVENTION, e.g. somatostatin or compound IVa, IVb or IVc, at a dosage rate of from ca. 0.001 to 2 mg/hour substantial reduction of pulmonary lesions induced by oxygen is observed in comparison with the control group. Thus somatostatin administered at a dosage of 1 mg/hour in the above test substantially reduces the oxygen toxicity. In particular, no significant lung exudation is observed in the animals treated with the COMPOUNDS OF THE INVENTION.

## B. Adult Respiratory Distress Syndrome

Five groups of guinea pigs (20 months, weight 750 to 1.150 g) are used for the study:

Group 1: Saline control group injected intravenously with 2 ml of saline.

Group 2: Septic control group injected intravenously with 2 x $10^9$ Escherischia coli.

Group 3: E. coli septicemia and a bolus of the compound to be tested intravenously injected 5 minutes before E. coli injection.

Group 4: E. coli septicemia and the compound to be tested in continuous infusion, begun with a bolus (same dose as in group 3) intravenously injected followed by continuous infusion, started 60 min before E. coli injection.

Group 5: Continuous infusion of the compound to be tested, begun with a bolus (same dose as in Group 3) intravenously injected, control.

At 1 hour intervals, mean arterial blood pressure is measured. At baseline and at 0.5, 1, 2, 4 and 8 hour 0.2 ml blood samples for total WBC, and differential courts are drawn. Arterial blood is collected in glass syringes and iced immediately and then the blood gases ($Pa_{o2}$, $Pa_{co2}$) and pH are determined on a blood gas analyzer. Airways resistance and compliance, minute ventilation, heart rate, oxygen consumption and lung volume are monitored serially for 8 hours. The animals are injected intravenously with 10 µCi of $^{125}$I-labelled albumin at 5 h and with 5 uCi of $^{51}$G-labelled erythrocytes in 3 ml of blood at 7 hour. To treat hypotension and dehydration during septicemia, 25 ml/kg of normal saline are administered intravenously to the five groups of animals hourly. After 8 h, the animals receive 30 mg of nembutal and 2,000 IU of heparin. Five minutes later the animals are killed; 8 ml of blood are drawn for preparation of plasma and are frozen in aliquots at - 20 ° C until needed. The chest is opened and each pulmonary hilus is double-clamped and transected between the clamps. The lungs are drained of free blood and the caudal lobes are placed in a tared container for determination of lung water.

The wet-to dry lung weight ratio is calculated to assess the lung water. Furthermore, the concentration ratio of $^{125}$I-labelled albumin in broncho alveolar lavage fluid to that in plasma (albumin index) and the accumulation of $^{125}$I-labelled albumin in the pulmonary interstitium and lymphatics (AIMS) are determined. Lung tissues are submitted to histological examination to assess neutrophil accumulation.

The COMPOUNDS OF THE INVENTION are active in this test when administered at a dosage in the range of from 0.01 µg/kg to 2 mg/kg i.v. Thus compound IVa administered in the form of a bolus of 100 µg/kg i.v. or in infusion at a dosage of from 0.01 µg/kg/hour attenuates lung injury induced by septic shock.

Equivalent results may be obtained in trials performed in relation to other diseases and conditions hereinbefore specified employing COMPOUNDS OF THE INVENTION, in particular compounds IVa, IVb or IVc, at equivalent dosage levels to those described above. A clinical trial may be carried out, e.g. with patients who have suffered irreversible brain damage receive ventilation with pure oxygen. The compound to be tested is administered s.c. or i.v. Measurements of arterial-blood gases, cardiac output, intrapulmonary shunt, ratio of dead space to tidal volume and lung-thorax compliance are made periodically. On administration of the COMPOUNDS OF THE INVENTION, for example compound IVa, IVb or IVc, at dosages of from 0.002 to 20 mg/day i.v., s.c. or by infusion, attenuation of oxygen toxicity is observed as compared with subjects receiving placebo.

Daily dosages required in practicing the method of the invention will vary depending upon, for example, the compound employed, the host, the mode of administration and the severity of the condition being treated. Doses may be in the range used to treat gastroenteropancreatic endocrine (GI) tumours such as vipomas, or acromegaly, to about 10 times that dose.

Thus for compound IVa GI tumours may be treated initially with 0.05 mg once or twice a day by subcutaneous injection. Dosage can be increased to 0.2 mg three times daily. For acromegaly daily doses of from 100 to 300 µg s.c. may be used. Compound IVa is tolerated at least to 1 mg.

Indicated daily doses for compound IVa in the method of the invention are from 0.025 to 1 mg, preferably 0.1 to 1 mg conveniently administered for example in divided doses up to four times a day or in sustained release form. COMPOUNDS OF THE INVENTION may be administered by any conventional route, in particular enterally, e.g. in the form of tablets or capsules, or preferably parenterally, e.g. in the form of injectable solutions or suspensions, e.g. s.c., i.m. or i.v. Compound IVa is preferably administered parenterally in the form of an injectable formulation, e.g. based on lactic acid. Compound IVa is the preferred compound. It is indicated that it may be administered at daily dosages of from 50 µg to 1 mg s.c. The compound IVb is preferably administered in an oral form, e.g. at a dosage of 2 µg to 20 mg p.o., preferably 300 to 5000 µg p.o.. Oral unit dosages may contain for example from about 0.5 µg to about 10 mg of compound IVb.

According to a further embodiment of the present invention, the COMPOUNDS OF THE INVENTION (in free form or pharmaceutically acceptable salt or complex form) may also be administered in the form of a pharmaceutical composition adapted for inhalation or insufflation. Such compositions may be liquid or in powdered form and may contain a pharmaceutically acceptable diluent or carrier. They may be administered as a powder, e.g. expelled from a powder blower or an insufflator, or as an aerosol or spray which distributes the active ingredient in the form of a powder or fine droplets of a suspension, solution or emulsion. In addition to the active ingredient, the aerosols or sprays may contain a propellant, e.g. a liquefied-gas propellant having a low boiling point and vapor pressure, and where required further pharmaceutically acceptable carriers, e.g. a liquid or solid non-ionic or anionic surfactant, and/or a pharmaceutically acceptable diluent and/or solvent and/or stabilizing or preserving agent. Aerosols may be produced by a manual spray process or from a pressurized package. The COMPOUNDS OF THE INVENTION may also be administered, e.g. in nebulized form from a solution, suspension or emulsion optionally containing a carrier, a diluent or a solvent, e.g. an aqueous saline, concomitant with oxygen therapy, e.g. in assisted respiration or mechanical ventilation procedures. Said administration may be in continuous flow or by intermittence.

The composition for use according to the invention may be prepared by bringing a COMPOUND OF THE INVENTION into intimate admixture with the pharmaceutically acceptable diluents or carriers and effecting formulation or presentation so as to provide for or permit convenient administration.

The intimate admixture may be performed at room temperature or at a higher temperature which does not impair the active ingredient. If required, a pharmaceutically acceptable emulsifying, surface active or preserving agent may be used.

The COMPOUNDS OF THE INVENTION may, if desired or as appropriate, be employed as adjunct or adjuvant to other therapy, e.g. a drug such as administered in the case of distress situations, for example glucocorticoid steroids, or monoclonal antibodies to LPS.

Where COMPOUNDS OF THE INVENTION are administered in conjunction with, e.g. as an adjuvant to, other therapy, e.g. for the treatment of specific diseases or conditions as hereinabove specified, dosages for the co-administered drug will of course vary depending on the type of drug employed, on the specific drug employed, on the condition to be treated, the therapy desired and so forth. In general however, satisfactory results may be obtained on administration of the co-administered drug at the same dosages as or at a dosage of the order of 80 % e.g. 50 % of those commonly required when the said co-administered drug is employed as mono-therapy. For example, hydrocortisone is administered in an emergency situation at a dose of from 500 mg to 1 g i.v. to adults.

In accordance with the foregoing the present invention provides, in a yet further aspect:

5. A method of co-administering a COMPOUND OF THE INVENTION and a glucocorticoid steroid in the prevention or treatment of ARDS to a subject in need of such a treatment or prevention.

The following is illustrative of the preparation of compositions in accordance with the invention.

**Concentration per ml**

**1. Ampoules**

| | Ex. 1 |
|---|---|
| A. Octreotide* | 0.05 mg |
| Mannitol | 45.0 mg |
| Lactic acid (88%) | 3.4 mg |
| Sodium hydrogenocarbonate | to pH 4.2 |
| Water(inject.grade) to 1 ml | |
| Carbon dioxide | q.s. |

* given as the acetate peptide hydrate content 87 per cent.

| | Ex. 2 |
|---|---|
| B. Octreotide* | 0.2 mg |
| NaCl | 7.5 mg |
| Lactic acid (88%) | 3.4 mg |
| Sodium hydrogeno-carbonate | to pH 4.2 |
| Water (injection grade) | to 1 ml |
| Carbon dioxide | q.s. |

* given as the acetate peptide hydrate content 87 per cent.

| **2. Vials** | |
|---|---|
| | Ex. 3 |
| Octreotide* | 0.2 mg |
| Mannitol | 45.0 mg |
| Lactic acid (88%) | 3.4 mg |
| Phenol | 5.0 mg |
| Sodium hydrogeno-carbonate | to pH 4.2 |
| Water (injection grade) | to 1 ml |
| Carbon dioxide | q.s. |

* given as the acetate peptide hydrate content 87 per cent.

The compositions comprising a somatostatin analogue or derivative, e.g. octreotide,may be prepared by standard techniques, e.g. in charges of 50 litres to provide about 43 000 ampoules of 1 ml or 8400 vials under carbon dioxide gassing. The compositions are filtered (e.g. through 0.2 micron holes at 0.5 bar) and introduced in the ampoules or vials under aseptic conditions.

COMPOUNDS OF THE INVENTION are well tolerated at dosages required for use in accordance with the present invention. Compounds IVa and IVb for example have the same order of tolerability in e.g. the mouse. For example an injection i.p. with 100 mg/kg no increase in GOT or any lethality is observed. For compound IVb for example, the maximal non-lethal dose in rats after 14 days p.o. is 2000 mg/kg body weight. Pharmaceutically acceptable salt forms exhibit the same or similar levels of tolerability/activity as the free compounds.

## Claims

1. A process for the preparation of a composition for use in protecting the lungs against acute lung injuries, said composition comprising i) native somatostatin or a somatostatin analogue or derivative in free form or in the form of a pharmaceutically acceptable salt or complex together with ii) a pharmaceutically acceptable diluent or carrier, which process comprises bringing component i) into intimate admixture with component ii) and effecting formulation or presentation so as to provide for or permit convenient administration.

2. A process according to claim 1, wherein component i) is a compound of formula I, II or III

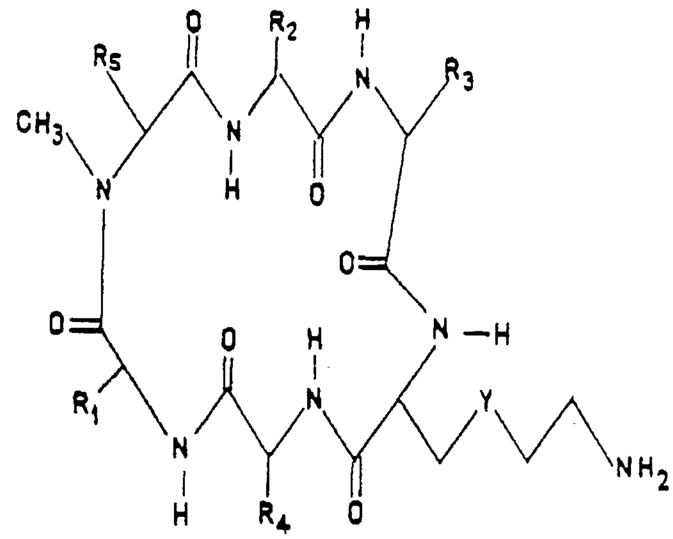

(I)

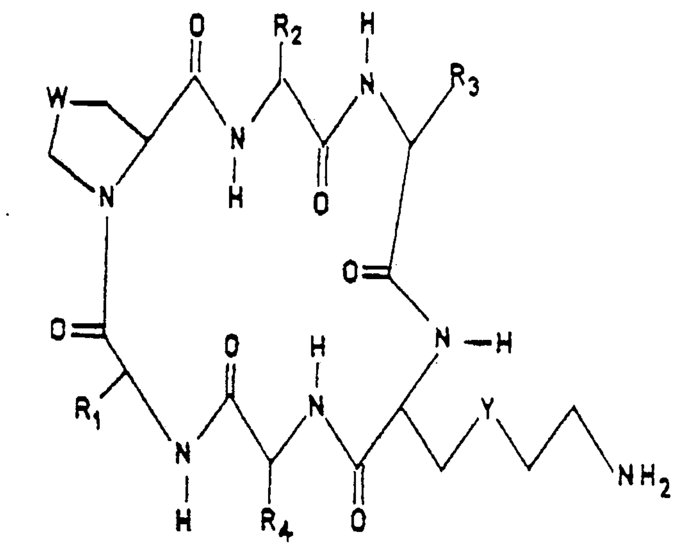

(II)

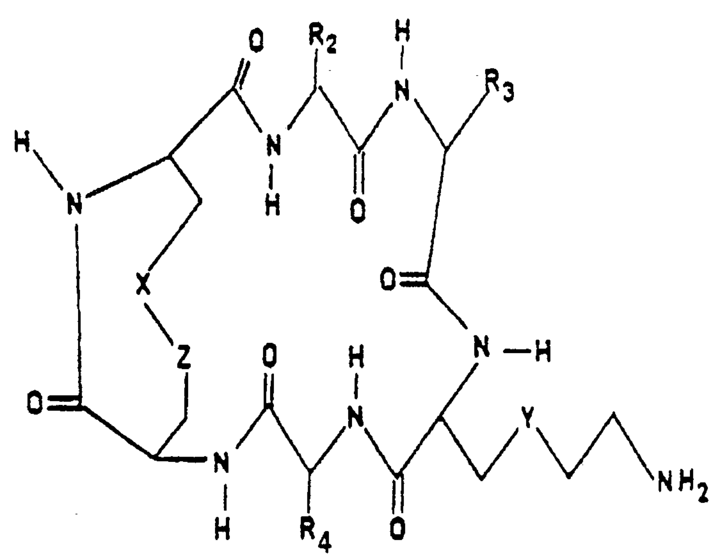

(III)

wherein
W is S or $(CH_2)_s$ where s is 0, 1 or 2;
one of X and Z is S and the other is S or $CH_2$;
Y is S or $(CH_2)_t$ where t is 0, 1 or 2;
each of $R_1$ and $R_2$ independently of the other, is $C_{1-5}$ alkyl, benzyl, benzyl having one or two $C_{1-5}$ alkyl, halogen, hydroxy, amino, nitro, and/or $C_{1-5}$ alkoxy substituents, or $C_{1-5}$ alkyl substituted with a 5- or 6-membered heterocyclic ring;
$R_3$ is 3-indolylmethyl, either unsubstituted or having $C_{1-5}$ alkyl, $C_{1-5}$ alkoxy or halogen substitution;
$R_4$ is $C_{1-5}$ alkyl, $C_{1-5}$ hydroxyalkyl, benzyl, carboxy-($C_{1-5}$ alkyl), amino ($C_{1-5}$ alkyl) or benzyl having a $C_{1-5}$ alkyl, halogen, hydroxy, amino, nitro and/or $C_{1-5}$ alkoxy substituent;
$R_5$ is $C_{1-5}$ alkyl, benzyl, or benzyl having a $C_{1-5}$ alkyl, halogen, hydroxy, amino, nitro, and/or $C_{1-5}$ alkoxy substituent,
in free form or in pharmaceutically acceptable salt or complex form.

3. A process according to claim 1, wherein component i) is a compound of formula IV

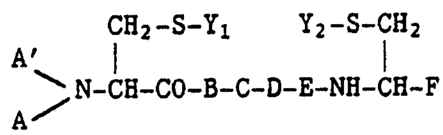

IV

wherein
A is $C_{1-12}$ alkyl, $C_{7-10}$ phenylalkyl or a group of formula RCO-, whereby
i) R is hydrogen, $C_{1-11}$ alkyl, phenyl or $C_{7-10}$ phenylalkyl, or
ii) RCO-is
a) an L- or D-phenylalanine residue optionally ring-substituted by halogen, $NO_2$, $NH_2$, OH, $C_{1-3}$ alkyl and/or $C_{1-3}$ alkoxy
b) the residue of a natural or a synthetic α-amino acid other than defined under a) above or of a corresponding D-amino acid, or
c) a dipeptide residue in which the individual amino acid residues are the same or different and are selected from those defined under a) and/or b) above,
the α-amino group of amino acid residues a) and b) and the N-terminal amino group of dipeptide residues c) being optionally mono-or di-$C_{1-12}$ alkylated or substituted by $C_{1-8}$alkanoyl,
A′ is hydrogen or, when A is $C_{1-12}$ alkyl or $C_{7-10}$ phenylalkyl, also $C_{1-12}$ alkyl or $C_{7-10}$ phenylalkyl,
$Y_1$ and $Y_2$ represent together a direct bond or each of $Y_1$ and $Y_2$ is independently hydrogen or a radical of formulae (1) to (5)

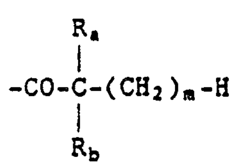

(1)

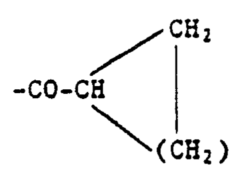

(2)

$-CO-NHR_c$

(3)

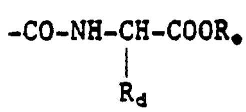

(4)

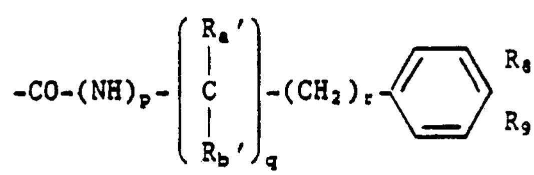

(5)

wherein $R_a$ is methyl or ethyl
$R_b$ is hydrogen, methyl or ethyl

m is a whole number from 1 to 4
n is a whole number from 1 to 5
$R_c$ is $(C_{1-6})$alkyl
$R_d$ represents the substituent attached to the α-carbon atom of a natural or synthetic α-amino acid (including hydrogen)
$R_e$ is $(C_{1-5})$alkyl
$R_a'$ and $R_b'$ are independently hydrogen, methyl or ethyl,
$R_8$ and $R_9$ are independently hydrogen, halogen, $(C_{1-3})$alkyl or $(C_{1-3})$alkoxy,
p is 0 or 1,
q is 0 or 1, and
r is 0, 1 or 2,
B is -Phe- optionally ring-substituted by halogen, $NO_2$, $NH_2$, OH, $C_{1-3}$alkyl and /or $C_{1-3}$alkoxy, or 3-(2-naphthyl)-alanine
C is (L)-Trp- or (D)-Trp- optionally α-N-methylated and optionally benzene-ring-substituted by halogen, $NO_2$, $NH_2$, OH, $C_{1-3}$ alkyl and/or $C_{1-3}$ alkoxy
D is Lys, Lys in which the side chain contains O or S in β-position, γF-Lys or δF-Lys, optionally α-N-methylated, or a 4-aminocyclohexylAla or 4-aminocyclohexylGly residue
E is Thr, Ser, Val, Phe, Tyr, Ile or an aminoisobutyric or aminobutyric acid residue
F is $-COOR_7$, $-CH_2OR_{10}$,

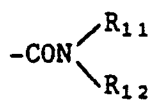 or 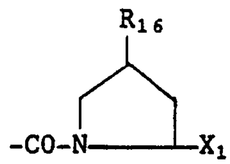

wherein
$R_7$ is hydrogen or $C_{1-3}$alkyl,
$R_{10}$ is hydrogen or the residue of a physiologically acceptable, physiologically hydrolysable ester,
$R_{11}$ is hydrogen, $C_{1-3}$alkyl, phenyl or $C_{7-10}$phenyl-alkyl, $R_{12}$ is hydrogen, $C_{1-3}$alkyl or a group of formula $-CH(R_{13})-X_1$,
$R_{13}$ is $CH_2OH$, $-(CH_2)_2-OH$, $-(CH_2)_3-OH$, or $-CH(CH_3)OH$ or represents the substituent attached to the α-carbon atom of a natural or synthetic α-amino acid (including hydrogen) and
$X_1$ is a group of formula $-COOR_7$, $-CH_2OR_{10}$ or

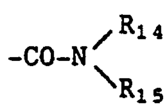

wherein
$R_7$ and $R_{10}$ have the meanings given above,
$R_{14}$ is hydrogen or $C_{1-3}$alkyl and
$R_{15}$ is hydrogen, $C_{1-3}$alkyl, phenyl or $C_{7-10}$phenylalkyl, and
$R_{16}$ is hydrogen or hydroxy,
with the proviso that
when $R_{12}$ is $-CH(R_{13})-X_1$ then $R_{11}$ is hydrogen or methyl,
wherein the residues B, D and E have the L-configuration, and the residues in the 2-and 7-position and any residues $Y_1$ 4) and $Y_2$ 4) each independently have the (L)- or (D)-configuration,
in free form or in pharmaceutically acceptable salt or complex form.

4. A process according to claim 3, wherein component i) is

**(D)Phe-Cys-Phe-(D)Trp-Lys-Thr-Cys-Thr-ol,**

in free form or in pharmaceutically acceptable salt or complex form.

5. A process according to claim 1, wherein component i) is a compound of formula IV as defined in claim 3 bearing at least one sugar residue on the N-terminal amino group.

6. A process according to any one of the preceding claims for the preparation of a composition for use in preventing or treating respiratory distress-syndrome.